Europäisches Patentamt

European Patent Office

Office européen des brevets

(11) Numéro de publication: **0 453 694 A1**

## DEMANDE DE BREVET EUROPEEN

(21) Numéro de dépôt: 90420209.0

(22) Date de dépôt: 27.04.90

(51) Int. Cl.⁵: **A61F 2/34**

(43) Date de publication de la demande:
30.10.91 Bulletin 91/44

(84) Etats contractants désignés:
AT BE CH DE DK ES FR GB GR IT LI LU NL SE

(71) Demandeur: **HIGH TECH INDUSTRIES - H.T.I.,
Société Anonyme dite:
11, Rue Jacques Monod
F-69680 Chassieu(FR)**

(72) Inventeur: **Gautier, Jean-Eugène
78, Rue Ferdinand Buisson
F-69003 Lyon(FR)**

(74) Mandataire: **Maureau, Pierre et al
Cabinet GERMAIN & MAUREAU B.P. 3011
F-69392 Lyon Cédex 03(FR)**

(54) **Cupule destinée à être fixée sans ciment pour prothèse totale de la hanche.**

(57) Cette cupule est du type composé de trois calottes sphériques (2, 3, 4), à savoir :
- une calotte extérieure (2) en matériau rigide dont la face externe est pourvue d'aspérités radiales (11) destinées à permettre sa fixation dans la cavité cotyloïdienne,
- une calotte intérieure (3) en matériau dur, biocompatible, à faible coefficient de frottement,
- et une calotte sphérique intermédiaire (4), en une matière semi-rigide; et dans la calotte extérieure (2) de laquelle sont découpées des languettes (5) mobiles radialement, en forme de portions de secteurs de calotte sphérique, définies par des fentes (5) en arc de cercle.

Les fentes (5) en arc de cercle s'étendent entre deux cercles parallèles au plan de l'ouverture (2b) de cette calotte, dont l'un est situé à proximité de ce plan et dont l'autre est situé près du sommet (2c) de la calotte, les extrémités de ces fentes (5) situées près du sommet (2c) de la calotte (2) étant, en outre, reliées, deux par deux, par des fentes (7) partiellement annulaires coïncidant avec celui des cercles précités situé près du sommet (2c) de la calotte (2).

Pour la pose de la calotte extérieure (2), on a conçu un appareil comportant des leviers (28) à crochets (31, 32), qui sont destinés à être engagés dans des encoches (33) et des lumières (34) dans les languettes (5,21).

FIG.2

## CUPULE DESTINEE A ETRE FIXEE SANS CIMENT POUR PROTHESE TOTALE DE LA HANCHE

La présente invention concerne une cupule destinée, à être fixée sans ciment, pour prothèse totale de la hanche.

Souvent, les cupules, actuellement fixées sans ciment outre les difficultés de pose qu'elles présentent notamment pour les vissées, ne donnent pas satisfaction car elles ne présentent pas une stabilité suffisante dans le temps

A l'origine, ces cupules étaient constituées d'une calotte sphérique exterieure en matériau rigide bio-compatible, tel qu'en acier inoxydable, portant, sur sa face externe, des moyens de fixation dans l'os de la cavité cotyloïdienne, tels que picots d'ancrage, filetage cylindrique ou conique, ou autre similaire, calotte extérieure dans la cavité intérieure de laquelle est fixée, de toute manière appropriée, une calotte sphérique intérieure en une matière, telle que le polyéthylène haute densité, présentant un faible coefficient de frottement par rapport au matériau constitutif de la tête céphalique de l'élément fémoral qui, généralement, est en acier inoxydable ou céramique.

Or, il est vraisemblable que le manque de stabilité dans le temps de ces cupules, provient essentiellement du fait que les efforts verticaux, directs ou résultants, sont transmis aux cupules sans amortissement par une couche élastique.

Pour tenter de remédier à cet inconvénient, on a conçu des cupules comportant une calotte sphérique intermédiaire en matériau souple et élastique, comme celles décrites dans le brevet européen 0066092 et dans les demandes de brevets PCT WO 86/02261 et européen 0253941. Cependant, dans ces cupules, ce sont les calottes sphériques interieures qui se sont révélées trop mobiles par rapport aux calottes sphériques externes fixées dans le cotyle, d'où il résultait des risques accrus de luxation.

Par ailleurs, les demandes de brevets européens 0169978 et 0242633 décrivent une cupule comportant deux éléments à savoir :
- une calotte sphérique extérieure en matériau rigide bio-compatible, tel qu'en acier inoxydable, dont la face externe est pourvue d'aspérités sensiblement radiales ou "picots" destinés à permettre sa fixation dans la cavité cotyloïdienne,
- une calotte sphérique intérieure en matériau bio-compatible, semi-rigide et, de préférence, élastique, telle qu'une matière plastique comme le polyéthylène, haute densité, cupule dont la calotte externe est rendue expansible par la présence de languettes en forme de portions de secteurs de calotte sphérique, séparées les unes des autres par des fentes en arcs de cercle, ayant une répartition angulaire régulière et susceptibles d'être déplacées radialement, vers l'extérieur, lors de la mise en place et de la fixation de cette calotte dans la cavité cotyloïdienne.

L'objectif visé par cette cupule était d'améliorer sa tenue dans le cotyle en provoquant l'expansion radiale de sa calotte externe après sa mise en place. Cependant, dans cette cupule, les fentes en arcs de cercle qui définissent les languettes radialement mobiles, n'atteignent pas le sommet de cette calotte et débouchent dans le bord périphérique de l'ouverture de sa cavité de telle sorte que les languettes ne sont tenues que par leur extrémité étroite voisine du sommet de la calotte et que, par conséquent, le diamètre de l'ouverture de la cavité de cette calotte peut se dilater lorsqu'elle est soumise à des efforts importants, et la répétition permanente de cette dilatation a évidemment pour effet d'ébranler peu à peu la cupule dont la tenue, dans le cotyle, se détériore progressivement.

En outre, la déformation de l'ouverture de cette cupule a pour inconvénient de favoriser le déscellement de la calotte dans la cavité cotyloïdienne. En effet, l'évasement diamétral sous l'effort, provoque par réaction le soulèvement de la calotte. De plus ce manque de rigidité diamétrale augmente les risques de luxation et interdit pratiquement l'utilisation d'une calotte intermédiaire en matériau suffisamment souple et élastique pour amortir les chocs et les efforts importants reçus par une calotte intérieure rigide.

La présente invention vise à remédier à tous ces inconvénients. A cet effet, dans la cupule qu'elle concerne, et qui est du type à 3 éléments, composé de trois calottes emboîtées l'une dans l'autre, dont celle extérieure, présente des languettes mobiles radialement, en forme de portions de secteurs de calotte sphérique, définies par des fentes en arcs de cercle, ayant une répartition angulaire régulière, les fentes en arcs de cercle s'étendent entre deux cercles parallèles au plan de l'ouverture de cette calotte, dont l'un est situé à proximité de ce plan et dont l'autre est situé près du sommet de la calotte, les extrémités de ces fentes situées près du sommet de la calotte étant, en outre, reliées, deux par deux, par des fentes partiellement annulaires coïncidant avec celui des cercles précités situé près du sommet de la calotte, de telle sorte que toutes les languettes précitées sont tenues par leur extrémité de grande dimension située près de l'ouverture de la calotte et que l'extrémité de petite dimension d'une languette, c'est-à-dire d'une portion sur deux de secteur de calotte sphérique, est déplaçable, radialement, vers l'intérieur ou l'extérieur, seules les languettes, mobiles radialement, portant, sur leur face externe, des picots d'ancrage.

Ainsi, les fentes en arcs de cercle qui délimitent les languettes de cette calotte ne débouchent pas dans le bord de son ouverture, de sorte que cette dernière ne peut pas être dilatée, ce qui procure à cette cupule, une excellente tenue dans le cotyle par sa ceinture diamétrale.

Il faut cependant remarquer que la course d'effacement quasiment nulle des extrémités des languettes les plus proches de l'ouverture de la cupule, s'oppose à la présence de picots près de cette extrémité des languettes.

Pour y remédier, suivant une forme d'exécution perfectionnée de cette cupule, visant à obtenir sensiblement une même valeur d'ancrage sur toute la surface externe de cette cupule, dans chaque languette liée à la calotte extérieure par son extrémité de grande dimension, la plus proche de l'ouverture de cette calotte, c'est-à-dire dans chaque languette principale, est découpée une languette secondaire inversée par rapport à la première, c'est-à-dire liée à cette dernière par son extrémité la plus proche du sommet de cette calotte dont l'extrémité libre est proche de l'ouverture de cette calotte et dont la face externe est couverte de picots d'ancrage jusqu'à proximité de son bord libre.

La présence des languettes secondaires permet donc, après mise en place de la calotte extérieure, de provoquer son expansion à proximité de son ouverture, ce que ne permet pas la seule présence des languettes principales et de lui procurer, ainsi, une meilleure fixation dans le cotyle.

En outre, la présence des languettes secondaires dont la face externe porte des picots d'ancrage qui sont susceptibles de s'effacer par une flexion de ces languettes secondaires en direction de l'intérieur de la calotte, permet une mise en place et un retrait faciles de cette cupule, malgré la présence de picots d'ancrage portés par les languettes secondaires, à proximité de la base des languettes principales, c'est-à-dire à proximité de l'ouverture de cette calotte extérieure, cette présence améliorant considérablement la tenue de cette cupule dans le cotyle.

Lors de la pose de cette cupule, sa calotte extérieure est parfaitement sphérique et ne présente aucun point saillant, toutes les languettes portant les picots étant en position rétractée par un système mécanique approprié. La mise en place de la calotte externe est aisée, puisqu'elle se déplace sans obstacle dans la cavité sphérique concordante pratiquée dans la cavité cotyloïdienne. Du fait de ce déplacement aisé, il est facile d'ajuster l'angle de la cupule avec précision. En fin d'engagement de cette calotte dans l'emplacement aménagé pour la recevoir, dans la cavité cotyloïdienne, ses languettes mobiles maintenues à l'intérieur sont alors libérées et mises en place par l'engagement, dans sa cavité, d'un outil d'impactage ou impacteur, avant que les calottes intermédiaire et intérieure, préassemblées, ne soient mises en place dans cette cavité.

Suivant une forme d'exécution simple de l'invention, les moyens pour la mise en place ou le retrait de cette cupule comprennent, d'une part, des encoches ou lumières méridiennes ménagées dans les languettes, principales et secondaires, à proximité de leur extrémité libre et, d'autre part, un appareil comportant, outre un manche tubulaire adaptable aux moyens traditionnels connus de visée et de réglage de l'angle d'orientation de la cupule, une tête en forme de plateau circulaire portant, sur sa face opposée à celle du manche, d'une part, un épaulement cylindrique de centrage de la calotte externe et présentant, à cet effet, le même diamètre que le bord intérieur de son ouverture et, d'autre part, des leviers à crochets dont les axes d'articulation orthogonaux à celui du manche leur permettent de pivoter entre une position effacée des crochets, c'est-à-dire rapprochée de l'axe du manche et une position active d'accrochage en fond d'encoche ou de lumière de languettes, c'est-à-dire éloignée de l'axe du manche, chaque levier portant deux crochets et les leviers et leurs crochets ayant la même répartition angulaire que les encoches ou lumières et chaque crochet étant lui-même disposé de manière à pouvoir être engagé, en position effacée, dans une encoche ou lumière d'une languette lorsque le levier qui le porte est déplacé dans un sens correspondant à son éloignement du plateau précité, des moyens étant prévus pour permettre de déplacer les axes d'articulation des leviers dans un sens ou dans l'autre, parallèlement à l'axe du manche, tandis que d'autres moyens sont prévus pour faire pivoter les leviers en position d'accrochage lorsqu'après leur engagement dans les encoches ou lumières, leurs axes d'articulation sont déplacés en direction du plateau, provoquant, de ce fait, une flexion de toutes les languettes en position effacée.

Cet appareil peut donc être utilisé aussi bien pour la mise en place de cette calotte externe que pour son retrait.

Suivant une forme d'exécution préférée de l'invention, les moyens pour déplacer les axes d'articulation des leviers à crochets dans les deux sens, parallèlement à l'axe du manche, comprennent, en combinaison, une bague supportant ces axes et montée, axialement mobile dans un évidement cylindrique d'une pièce cylindrique coaxiale au plateau et présentant des encoches radiales dont chacune sert au logement et au guidage d'un levier à crochets, une tige de commande coaxiale au plateau et au manche dans l'alésage duquel elle est logée, dont une extrémité est liée, axialement, par goupille ou autre similaire, à la bague supportant les axes d'articulation des leviers à crochets et dont l'autre extrémité est liée, axialement, à un anneau fileté monté dans un alésage taraudé d'un volant de manoeuvre porté par le manche, cet anneau fileté étant, en outre, lié en rotation au manche, par exemple par une goupille traversant l'anneau fileté, l'extrémité considérée de la tige de commande et des lumières longitudinales diamétralement opposées du manche tubulaire.

Ainsi, pour déplacer les axes d'articulation des leviers parallèlement à l'axe du manche, il suffit de faire pivoter le volant de manoeuvre dans le sens correspondant à la direction du déplacement désiré, tout en tenant le manche pour l'empêcher de pivoter.

Par ailleurs, suivant une forme d'exécution simple de l'invention, les moyens pour faire pivoter les leviers à crochets, de leur position effacée à leur position d'accrochage, comprennent, d'une part, un bossage porté par chaque fond d'encoche radiale servant au logement et au guidage d'un levier à crochets auquel est associé un bossage opposé porté par la face la plus interne radialement de chaque levier à crochets, ces bossages étant disposés de manière que le second monte sur le premier dès que, depuis leur position la plus éloignée du plateau, les axes d'articulation des leviers sont déplacés en direction du plateau et, d'autre part, un moyen à ressort annulaire entourant les leviers à crochets et agissant sur eux pour tendre constamment à les ramener en position effacée.

Avec cet appareil, lorsque la calotte externe est correctement positionnée dans le cotyle, il suffit alors de faire tourner le volant de manoeuvre en sens inverse pour libérer toutes les languettes et les laisser reprendre leur position d'origine. Cependant, la résistance que leurs picots rencontrent dans le cotyle n'autorise pas leur retour à leur position d'origine, de sorte qu'il est nécessaire qu'au fur et à mesure de ce retour, le praticien cesse de faire pivoter le volant de manoeuvre et applique, sur le manche, par exemple à l'aide d'une masselotte, quelques chocs dirigés axialement.

Suivant une caractéristique préférée de l'invention, visant à réduire le temps de pose de cette cupule, la calotte intermédiaire est fixée à la calotte extérieure par un système d'accrochage à baïonnette ou par encliquetage d'un bourrelet dans une gorge, permettant une fixation plus rapide et plus aisée qu'avec un filetage, et la calotte intérieure est fixée à la calotte intermédiaire par encliquetage.

Il faut noter que la composition de cette cupule permet le maintien constant d'un contact intime non seulement entre ses trois calottes mais aussi avec la cavité cotyloïdienne, ce qui élimine pratiquement tout risque de déplacement et garantit une excellente répartition des charges. En effet, toute poussée appliquée aux languettes d'ancrage provoque, par réaction, l'appui de la calotte dans le fond de la cavité cotyloïdienne.

De toute façon, l'invention sera bien comprise à l'aide de la description qui suit en référence aux dessins schématiques annexés, représentant, à titre d'exemple non limitatif, une forme d'exécution de cette cupule :
- Figure 1 en est une vue en plan par-dessus ;
- Figure 2 en est une vue éclatée de côté en coupe axiale ;
- Figure 3 en est une vue en coupe suivant 3-3 de figure 1 montrant la calotte extérieure, seule, après son introduction dans la cavité cotyloïdienne ;
- Figure 4 montre la cupule complète après fixation dans la cavité cotyloïdienne ;
- Figure 5 est, et à échelle agrandie, une vue partielle en coupe suivant 5-5 de figure 2.
- Figure 6 en est une vue en plan par-dessus similaire à figure 1, illustrant une variante d'exécution de cette cupule ;
- Figure 7 est une vue en coupe suivant VII-VII de figure 6, montrant toutes les languettes en position effacée ;
- Figure 8 est une vue en perspective d'un appareil pour la mise en place ou le retrait de la calotte externe de cette cupule ;
- Figure 9 est une vue en coupe axiale de l'appareil de figure 8, avec les languettes en position normale ;
- Figure 10 et 11 sont des vues partielles en coupe, montrant la tête de l'appareil d'impaction en deux positions successives de mise en position effacée des languettes.

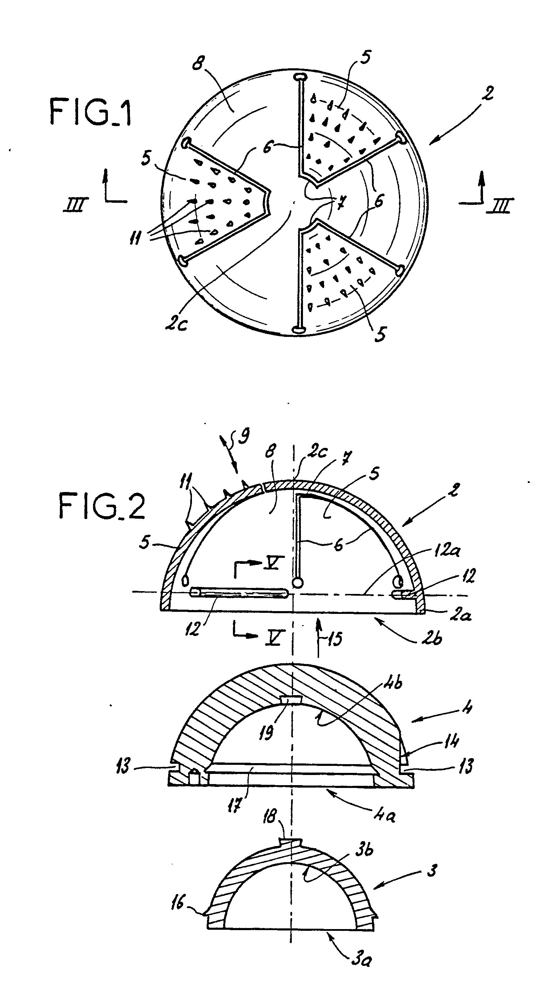

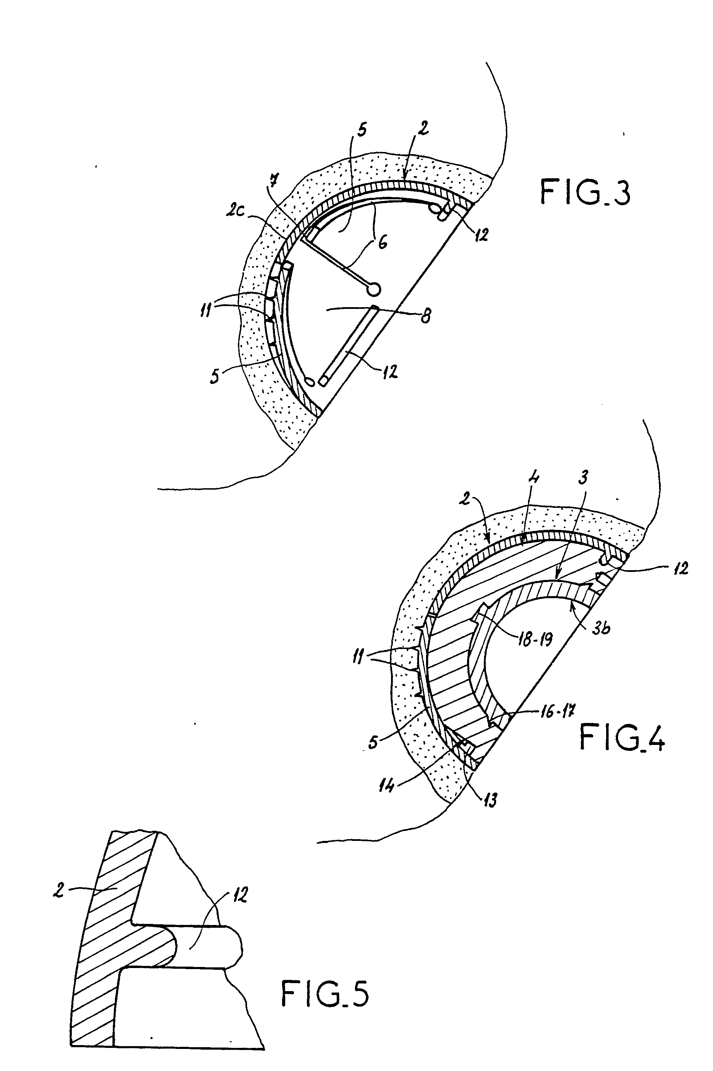

Comme le montrent plus particulièrement les figures 2 et 4, la cupule selon l'invention est du type constitué de 3 éléments à savoir :
- une calotte sphérique extérieure 2 en matériau rigide bio-compatible, tel qu'en acier inoxydable ou en titane, destinée à être ancrée dans la cavité cotyloïdienne du patient,
- une calotte sphérique intérieure 3 en matériau dur bio-compatible, tel qu'en céramique d'alumine ou de zircone ou autre similaire, dont au moins la face intérieure 3b présente un faible coefficient de frottement par rapport au matériau constitutif de la tête céphalique de l'élément fémoral associé à cette cupule et qui est généralement en céramique convenablement choisie,
- une calotte sphérique intermédiaire 4 en une matière souple telle qu'en polyéthylène à basse densité, et qui est destinée à jouer le rôle d'amortissement entre la calotte intérieure 3 et la calotte extérieure, pour éviter la transmission brutale, à cette dernière, des efforts verticaux importants susceptibles de l'ébranler jusqu'à détérioration de la qualité de sa fixation.

La calotte extérieure 2 présente des languettes 5 mobiles radialement, en forme de portions de secteurs de calotte sphérique et dont chacune est délimitée par deux fentes en arcs de cercle 6, de répartition angulaire régulière et dont les extrémités sont situées, respectivement, près du bord 2a de l'ouverture 2b de cette calotte, et près de son sommet 2c. Les extrémités des deux fentes 6, délimitant une même languette 5 et situées près du sommet 2c de la calotte 2 sont, en outre, reliées par une fente 7 partiellement annulaire située sur le même cercle que les fentes 7 des autres languettes 5, ce cercle étant concentrique au sommet 2c de la calotte 2.

Comme le montre plus particulièrement la figure 1, chaque languette 5 de la calotte 2, qui dans cet exemple en comporte trois, est liée à la calotte 2 par son extrémité de grande dimension située près de l'ouverture 2b de la calotte. On peut noter que deux languettes 5, adjacentes, sont séparées l'une de l'autre par une portion de secteur de calotte sphérique 8 similaire aux languettes 5 mais non mobile, ayant pour effet de conserver, à la calotte 8, une rigidité suffisante pour empêcher sa déformation.

Chaque languette 5, qui est apte à pivoter autour de son grand côté ou de sa grande base, comme indiqué par la double flèche 9 de figure 2, porte, sur sa face externe, des picots d'ancrage 11 orientés sensiblement radialement, c'est-à-dire suivant le rayon de rotation de la languette qui a pour axe de pivotement son bord adjacent à l'ouverture 2b de cette calotte 2. La disposition des picots et leur forme pyramidale favorisent leur pénétration suivant ce rayon dans la cavité cotyloïdienne.

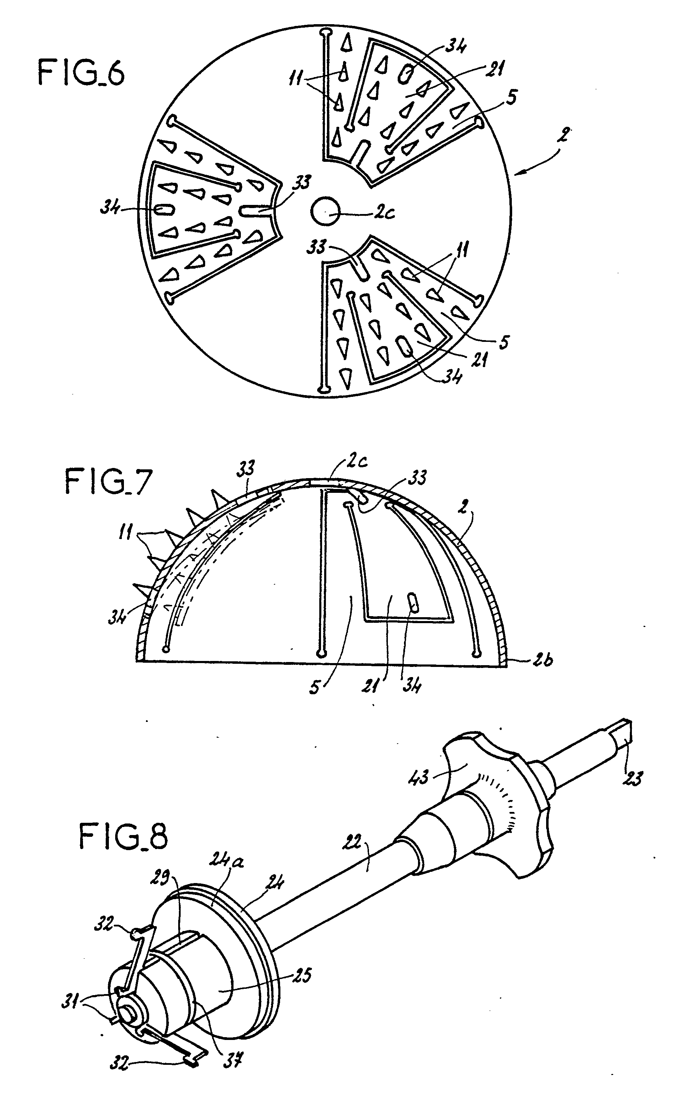

Les figures 2 et 5 montrent aussi une forme d'exécution avantageuse des moyens d'assemblage de la calotte intermédiaire 4 à la calotte extérieure 2. A cet effet, la calotte 2 porte, sur sa face interne et à proximité du bord de son ouverture 2b, trois segments de nervure 12 appartenant à un même cercle 12a parallèle au plan de l'ouverture 2b et dont chacun est destiné à constituer l'un des crans d'accrochage mâles d'un système d'accrochage du type à baïonnette. Chaque segment de nervure 12 s'étend sur un arc de cercle inférieur à 60°.

De son côté, la calotte intermédiaire 4 présente, sur sa face externe, 3 segments de rainure 13 ayant la même répartition angulaire régulière que les segments de nervure 12, mais de longueur sensiblement égale au double. Une encoche d'entrée 14 est ménagée à chacune des extrémités se correspondant de chacune des gorges 13, pour permettre l'engagement de chacune d'elles sur l'une des nervures 12 par un mouvement d'introduction axial, c'est-à-dire dans le sens de la flèche 15, suivi d'un mouvement de rotation autour de son rayon perpendiculaire au plan de son ouverture 4a.

Ce système d'accrochage ou d'assemblage, du type à baïonnette, présente l'avantage d'être d'une mise en oeuvre beaucoup plus rapide que tout autre système connu par filetage cylindrique ou conique qui nécessite évidemment d'effectuer plusieurs tours complets de rotation pour obtenir l'assemblage.

La figure 2 montre aussi le mode d'assemblage de la calotte intérieure 3 à la calotte intermédiaire 4 et qui est du type encliquetage. A cet effet, d'une part, la calotte intérieure 3 présente une nervure périphérique 16 circulaire, parallèle au plan de son ouverture 3a, tandis que la calotte intermédiaire 4 présente, dans sa cavité sphérique 4b, une gorge circulaire périphérique 17, de section transversale complémentaire de celle de la nervure 16, l'engagement de la nervure 16 dans la rainure 17 étant facilité par l'élasticité de la matière constitutive de la calotte 4. On conçoit aisément que l'assemblage de ces calottes intérieure 3 et intermédiaire 4 peut aussi être réalisé de manière instantanée.

Pour améliorer la liaison mécanique entre la calotte intérieure 3 et la calotte intermédiaire 4, il est possible de prévoir, en outre, comme le montre la figure 2 sur le sommet de la face externe de la calotte intérieure 3, un téton 18 engageable dans un logement 19, ménagé pour le recevoir, au sommet ou pôle de la cavité 4b de l'élément intermédiaire 4.

Il est indiqué précédemment que la calotte intérieure 3 présente une cavité intérieure 3b offrant un très faible coefficient de frottement au matériau constitutif de la tête céphalique de l'élément fémoral. A cet effet, cette cavité intérieure 3b est avantageusement constituée d'une céramique d'alumine, de zircone ou de tout autre céramique appropriée.

La conception de cette cupule montre à l'évidence que, grâce à la constitution de sa calotte extérieure 2, elle présente un diamètre d'ouverture 2b pratiquement indéformable, ce qui diminue considérablement tout risque de mouvement relatif de cette calotte extérieure 2 par rapport à la cavité cotyloïdienne, dans laquelle elle est fixée, ainsi que le risque d'une luxation.

Pour permettre la pose et le retrait de cette cupule sans endommager le cotyle, comme le montre très bien la figure 1, il est préférable de ne pas prévoir de picots 11 au voisinage de la base de chaque languette 5, c'est-à-dire à proximité de son extrémité par laquelle elle est liée à la calotte extérieure 2. En effet, du fait de sa liaison avec la calotte 2, cette partie de chaque languette 5 ne peut subir qu'un mouvement de basculement de faible amplitude, de sorte que si elle était couverte de picots, ces derniers ne seraient pas effaçables et gêneraient le mise en place de la calotte. Il en résulte qu'à proximité de son ouverture, l'ancrage de la calotte extérieure dans le cotyle n'est pas excellent. Pour y remédier, suivant une variante d'exécution perfectionnée de l'invention, dans chaque languette principale 5 est découpée une languette secondaire 21, inversée par rapport à la première, chaque languette secondaire 21 étant liée à la languette principale 5, qui la porte par son extrémité la plus proche du sommet 2c de la calotte externe 2.

En outre, l'extrémité libre de chaque languette secondaire 21 est située au voisinage du bord libre de cette calotte 2, entourant son ouverture 2b.

Comme le montre plus particulièrement la figure 7, la présence de ces languettes secondaires 21 susceptibles d'être fléchies en direction de l'intérieur de la calotte externe 2 par pivotement autour de leur base ou extrémité la plus proche du sommet 2c de cette calotte, permet d'effacer les picots 11 qu'elles portent au voisinage de leur extrémité libre, c'est-à-dire au voisinage de l'ouverture 2b de la calotte 2 et qui, de ce fait, ne gênent pas la mise en place de la calotte 2. Par la suite, après impaction, l'engagement de ces picots dans le cotyle améliore considérablement la tenue de la calotte externe 2, et par conséquent de la cupule, dans la cavité cotyloïdienne du patient.

La pose de cette calotte externe dans la cavité cotyloïdienne nécessite donc d'amener préalablement les languettes principale 5 et secondaire 21 en position effacée, comme illustré sur la figure 7, de manière à ne pas être gêné par la présence des picots d'ancrage 11 sur leur face extérieure. A cet effet, on peut utiliser l'appareil représenté sur les figures 8 à 11, spécialement conçu pour cette opération.

Comme le montrent ces figures, cet appareil comprend essentiellement un manche tubulaire 22, dont l'extrémité postérieure 22a porte un tenon 23 permettant son adaptation à un appareil d'un type connu de visée et de réglage de l'angle d'orientation de la calotte externe 2 dans le cotyle du patient.

A son extrémité antérieure 22b, le manche tubulaire 22 porte une tête en forme de plateau circulaire 24.

Sur sa face opposée à celle du manche 22, le plateau 24 présente un épaulement cylindrique 24a destiné au centrage de la calotte externe 2 et présentant, à cet effet, le même diamètre que le bord intérieur de son ouverture 2b.

Sur cette même face, le plateau 24 porte une pièce cylindrique 25 coaxiale à lui-même et au manche tubulaire 22, qui lui est fixé par engagement dans un alésage central 24b, qu'il présente pour la recevoir.

Cette pièce cylindrique 25 présente, à son extrémité engagée dans l'alésage 24b du plateau 24, un évidement cylindrique axial 25a servant au logement, avec possibilité de déplacement axial d'une bague 26 portée par le manchon tubulaire 22.

La différence entre la longueur de la bague 26 et celle de son logement 25a, détermine sa course axiale.

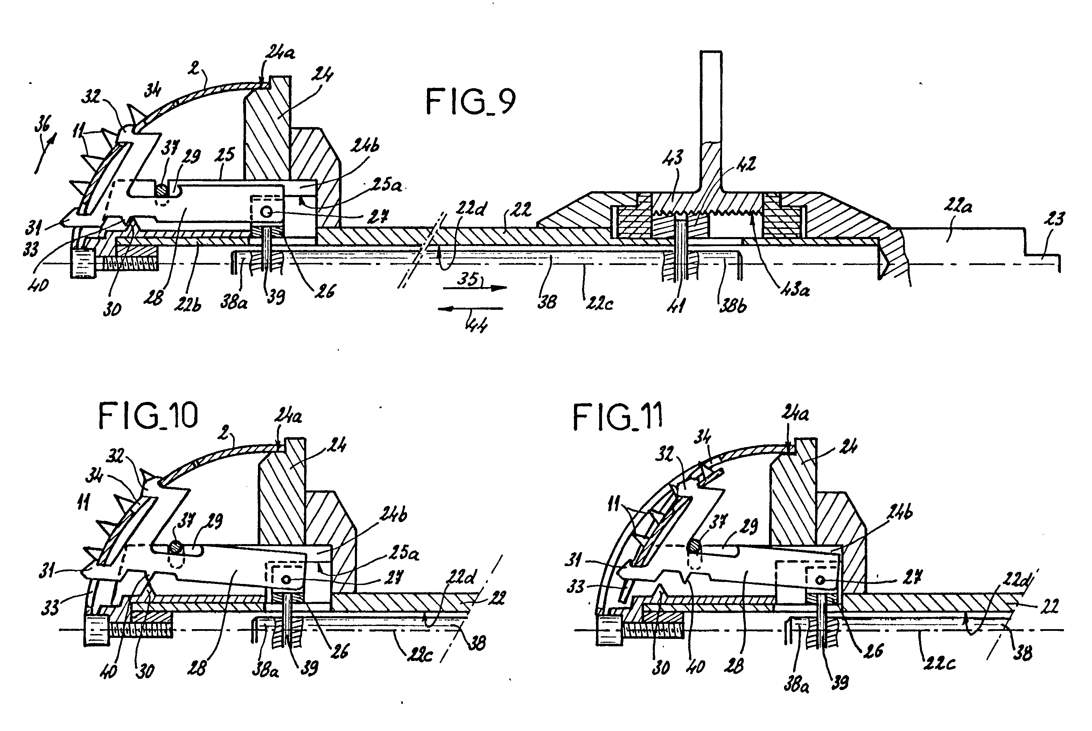

Cette bague 26 porte trois axes 27, dont un seul est visible sur les figures 9 à 11, et qui sont disposés orthogonalement au manche 22, avec une répartition angulaire régulière autour de ce manche.

Sur chaque axe 27, est articulé un levier 28 normalement parallèle à l'axe 22c du manche tubulaire 22 et orienté dans la direction opposée à celle du manche tubulaire 22.

Chaque levier 28 est logé dans une encoche radiale 29, ménagée pour le recevoir, dans la pièce cylindrique 25.

Chaque levier 28 présente, à son extrémité opposée à celle par laquelle il est articulé à l'axe correspondant 27, deux crochets, respectivement 31 et 32, dont le bec d'accrochage est orienté radialement vers l'extérieur par rapport à l'axe 22c du manche tubulaire 22. Chaque crochet 31 et 32 est destiné à être engagé, respectivement, dans une encoche 33 et une lumière 34 prévues pour le recevoir, respectivement, dans chaque languette principale 5 et dans chaque languette secondaire 21, et dont le mode d'utilisation sera décrit ultérieurement.

Ces encoches 33 et lumières 34 sont centrées sur le méridien central des languettes correspondantes 5 et 21.

Chaque fond d'encoche 29 présente un bossage 30 dirigé radialement vers l'extérieur et chaque levier 28 présente, sur sa face tournée vers le fond de l'encoche 29 lui servant de logement, un bossage inverse 40, ces deux bossages 30 et 40 étant disposés de manière que lorsque les leviers 28 sont en position la plus avancée à l'extérieur, c'est-à-dire dans la position correspondant à la fin de course de la bague 26 contre le fond de son logement 25a le plus éloigné de l'extrémité postérieure 22a du manche tubulaire 22, comme illustré sur la figure 9, le bossage 40 porté par chaque levier 28 soit disposé juste en avant du bossage 30 porté par le fond de l'encoche 29 correspondante.

On conçoit aisément qu'un déplacement axial de la bague 26 en direction du fond opposé de son logement 25a, c'est-à-dire dans le sens de la flèche 35, a pour effet de faire monter les bossages 40 des leviers 28 sur les bossages 30 des fonds de leurs encoches 29 et par conséquent, de faire pivoter chaque levier 28 radialement vers l'extérieur, c'est-à-dire dans le sens de la flèche 36 pour le levier 28 visible sur les figures 9 à 11.

Ce pivotement, qui n'est provoqué que par une très faible fraction de la course axiale de la bague 26, dans le sens de la flèche 35, provoque l'engagement des crochets 31 et 32, respectivement sur le fond d'encoche 33 et sur le fond de lumière 34, de telle sorte que chaque crochet 28, par la poursuite de son déplacement dans le sens de la flèche 35, provoque le cintrage des languettes principale 5 et secondaire 21 correspondantes, en direction de l'intérieur de la calotte externe 2, comme illustré sur les figures 10 et 11, jusqu'à ce que leurs picots 11 soient totalement effacés par rapport à la face externe de la calotte 2, c'est-à-dire dans la position illustrée par la figure 11.

Comme le montrent les figures 10 et 11, l'engagement des crochets 31 et 32 dans les fonds d'encoches 33 et de lumières 34, combiné à la flexion des languettes principale 5 et secondaire 21, rend impossible tout désengagement intempestif des crochets, malgré la présence d'un ressort annulaire 37 entourant les leviers 28 et tendant, constamment, à les ramener dans leur position d'origine parallèle à l'axe 22c du manche tubulaire 22.

Il est en effet indispensable que les leviers 28 soient constamment rappelés dans leur position parallèle à l'axe 22c du manche tubulaire 22, pour que leurs crochets 31 et 32 soient aptes à être engagés dans les encoches 33 et lumières 34, malgré, notamment, l'action de la pesanteur tendant à faire pivoter certains d'entre eux dans le sens illustré par la flèche 36.

Dans l'exemple illustré sur les figures 8 à 11, les moyens d'actionnement de la bague 26, dans le sens de la flèche 35 et en sens inverse, sont constitués par une tige de commande 38, logée dans l'alésage 22d du manche tubulaire 22, dont l'extrémité antérieure 38a est liée axialement, par une goupille 39, à la bague 26 et dont l'extrémité postérieure 38b est liée axialement, par une goupille 41, à un anneau fileté 42 monté dans un alésage taraudé 43a d'un volant de manoeuvre 43 porté par le manche tubulaire 22.

Le mode d'utilisation de cet appareil est donc le suivant :

Le volant de manoeuvre 43 ayant été pivoté par le praticien dans le sens correspondant au déplacement de l'anneau fileté 42 et de la bague 26, dans le sens opposé à celui de la flèche 35, c'est-à-dire dans le sens de la flèche 44, les leviers 28 et leurs crochets 31 et 32 sont amenés dans leur position la plus avancée.

Le praticien peut alors engager la calotte externe 2 sur les leviers 28, jusqu'à ce que d'une part, chaque crochet 31 et 32 saille au-delà des languettes, respectivement 5 et 21, à travers les encoches 33 et les lumières 34 et d'autre part, que le bord périphérique de son ouverture 2b soit centré par rapport au plateau 24, par son engagement sur l'épaulement 24a de ce dernier.

Tout en maintenant la calotte 2 dans cette position, le praticien peut manoeuvrer le volant 43 en sens inverse, pour déplacer l'anneau fileté 42 et la bague 26 en sens inverse, c'est-à-dire dans le sens de la flèche 35.

Comme indiqué précédemment lors du déplacement des leviers 28 dans le sens de la flèche 35, et par suite de leur pivotement radialement vers l'extérieur, leurs crochets 31 et 32 s'engagent sur les fonds d'encoches 33 et de lumières 34 des languettes principale 5 et secondaire 21, comme indiqué précédemment et comme illustré sur les figures 10 et 11. La poursuite de ce déplacement des leviers 28 dans le sens de la flèche 35, provoque donc le cintrage des languettes 5 et 21.

Les picots 11 des languettes principale 5 et secondaire 21 étant alors amenés en position effacée, comme illustré sur la figure 11, le praticien peut alors monter l'appareil, par le tenon 23 de son extrémité postérieure 22a, sur un appareil de visée et de guidage lui permettant de positionner très correctement la calotte 2 dans la cavité cotyloïdienne du patient.

Cette opération se fait d'autant plus aisément que tous les picots 11 sont effacés.

La calotte 2 étant dans sa position angulaire correcte, il faut alors que le praticien manoeuvre à nouveau le volant 43 en sens inverse de celui indiqué précédemment pour faire déplacer l'anneau fileté 42, la bague 26 et les leviers 28 en sens inverse, c'est-à-dire dans le sens illustré par la flèche 44.

Il en résulte que, par leur déplacement dans ce sens, les leviers 28 et leurs crochets 31 et 32 libèrent progressivement les languettes principale 5 et secondaire 21, qui ont alors tendance à reprendre leur position d'origine, illustrée sur la figure 9, par suite de l'élasticité de leur matière constitutive.

Etant donné que leurs picots 11 rencontrent une résistance au contact du cotyle du patient, la manoeuvre du volant 43 sera freinée, la course de toutes les languettes, 5 et 21, en direction de leur position de repos, étant empêchée par cette résistance. Il faudra alors que le praticien applique sur l'appareil des chocs, par exemple à l'aide de la masselotte, dont l'ensemble de visée et de réglage est généralement équipé, chocs qui forceront les picots 11 à pénétrer dans le cotyle.

En répétant plusieurs fois, successivement, la manoeuvre du volant 43 dans le sens dernièrement précité, et celle de la masselotte, le praticien pourra obtenir la fixation totale de la calotte 2 dans le cotyle.

Il est bon de remarquer que le sens de pivotement des languettes principales et la pénétration de leurs picots 11 dans le cotyle, a pour effet d'exercer sur la calotte 2 une traction orientée dans le sens de la flèche 44, qui assure une excellente application de la calotte 2 contre le cotyle.

Naturellement, par des manoeuvres similaires à celles précitées, il sera possible de retirer, sans aucune difficulté, une calotte 2 du cotyle d'un patient, si par exemple cette calotte doit être remplacée, par suite d'une quelconque détérioration, et notamment par suite d'une détérioration du cotyle de ce patient.

La calotte intermédiaire 4 est ensuite engagée et fixée dans la calotte extérieure 2, de préférence après que la calotte 3 lui ait été préassemblée. Naturellement, la mise en place de la calotte intermédiaire 4 verrouille les languettes 5 de la calotte extérieure 2, les empêchant de basculer en direction de l'intérieur de cette calotte, ce qui contribue à en assurer le maintien en position d'ancrage.

Etant donné que la forme extérieure de la calotte extérieure 2, après pose de la cupule, correspond à la forme qu'elle occupe, naturellement avant sa pose, il est tout à fait possible et facile d'aménager, dans la cavité cotyloïdienne du patient, un berceau de forme complémentaire contre lequel la calotte extérieure 2 de cette cupule sera appliquée avec contact intime.

Ce caractère indéformable du diamètre de l'ouverture 2b de la calotte extérieure 2 permet d'utiliser, sans risque de déplacement intempestif ni de luxation, une calotte intermédiaire 4 possédant des qualités relativement importantes de souplesse et d'élasticité qui ont pour effet d'améliorer considérablement la fonction amortisseur de cette calotte intermédiaire 4 par rapport aux cupules antérieures connues. La souplesse de cette calotte intermédiaire 4 contribue aussi à assurer un contact intime entre les trois éléments de cette cupule, de telle sorte qu'on obtient ainsi une excellente répartition des contraintes transmises par le fémur à la hanche du patient.

**Revendications**

1. Cupule destinée à être fixée sans ciment pour prothèse totale de la hanche du type composé de trois calottes sphériques (2, 3, 4) emboîtées l'une dans l'autre, à savoir :
- une calotte sphérique extérieure (2) en matériau rigide bio-compatible, tel qu'en acier inoxydable, ou titane, dont la face externe est pour vue d'aspérités radiales (11) ou "picots" destinés à permettre sa fixation dans la cavité cotyloïdienne ;
- une calotte sphérique intérieure (3) en matériau dur, bio-compatible, à faible coefficient de frottement par rapport au matériau constitutif de la tête céphalique, tel que, par exemple, une céramique d'alumine ou autre similaire ;
- et une calotte sphérique intermédiaire (4), en une matière semi-rigide et de préférence élastique, telle qu'une matière plastique comme le polyéthylène, et dans la calotte extérieure (2) de laquelle sont découpées dans languettes (5) mobiles radialement, en forme de portions de secteurs de calotte sphérique, définies par des fentes (5) en arcs de cercle, ayant une répartition angulaire régulière, cupule caractérisée en ce que les fentes (5) en arcs de cercle s'étendent entre deux cercles parallèles au plan de l'ouverture (2b) de cette calotte, dont l'un est situé à proximité de ce plan et dont l'autre est situé près du sommet (2c) de la calotte, les extrémités de ces fentes (5) situées près du sommet (2c) de la calotte (2) étant, en outre, reliées, deux par deux, par des fentes (7) partiellement annulaires coïncidant avec celui des cercles précités situé près du sommet (2c) de la calotte (2), de telle sorte que toutes les languettes (5) sont tenues par leur extrémité de grande dimension située près de l'ouverture (2b) de la calotte (2) et que l'extrémité de petite dimension d'une languette (5), c'est-à-dire d'une portion sur deux de secteur de calotte sphérique, est déplaçable, radialement, vers l'intérieur ou l'extérieur, seules les languettes (5) mobiles radialement, portant, sur leur face externe, des picots d'ancrage (11).

2. Cupule selon la revendication 1, caractérisée en ce que, dans chaque languette (5), liée à la calotte extérieure (2) par son extrémité la plus proche de l'ouverture (2b) de cette calotte (2), c'est-à-dire dans chaque languette principale, est découpée une languette secondaire inversée (21), c'est-à-dire liée à la languette principale (5), par son extrémité la plus proche du sommet (2c) de la calotte extérieure (2) et dont l'extrémité libre est proche du bord libre entourant l'ouverture (2b) de cette calotte (2), la face externe de chaque languette secondaire (21) étant couverte de picoits d'ancrage (11) jusqu'au voisinage de son extrémité libre.

3. Cupule selon la revendication 1 ou 2, caractérisée en ce que la calotte intermédiaire (4) est fixée à la calotte extérieure (2) par un système d'accrochage à baïonnette (12, 13, 14) permettant un assemblage très rapide.

4. Cupule selon l'une des revendications 1 à 3, caractérisée en ce que la calotte intérieure (3) est fixée à la calotte intermédiaire (4) par encliquetage d'une nervure circulaire (16), dans une gorge (17) également circulaire, et par l'engagement d'un têton (18) dans une cavité conique (19) évitant tout mouvement relatif des surfaces sphériques en présence.

5. Moyens pour la mise en place, dans le cotyle d'un patient, d'une cupule selon l'une quelconque des revendications 1 à 4, ou son retrait, caractérisé en ce qu'ils comprennent, d'une part, des encoches (33) ou lumières (34) méridiennes ménagées dans les languettes, principale (5) et secondaire (21), à proximité de leur extrémité libre et, d'autre part, un appareil comportant, outre un manche tubulaire (22) adaptable aux moyens traditionnels connus de visée et de réglage de l'angle d'orientation de la cupule, une tête en forme de plateau circulaire (24) portant, sur sa face opposée à celle du manche (22), d'une part, un épaulement cylindrique (24a) de centrage de la calotte externe (2) et présentant, à cet effet, le même diamètre que le bord intérieur de son ouverture (22) et, d'autre part, des leviers (28) à crochets (31, 32) dont les axes d'articulation (27) orthogonaux à celui (22c) du manche (22) leur permettent de pivoter entre une position effacée des crochets (31, 32), c'est-à-dire rapprochée de l'axe (22c) du manche (22) et une position active d'accrochage en fond d'encoche (33) ou de lumière (34) de languettes (5, 21), c'est-à-dire éloignée de l'axe (22c) du manche (22), chaque levier (28) portant deux crochets (31, 32) et les leviers (28) et leurs crochets (31, 32) ayant la même répartition angulaire que les encoches (33) ou lumières (34) et chaque crochet (31, 32) étant lui-même disposé de manière à pouvoir être engagé, en position effacée, dans une encoche (33) ou lumière (34) d'une languette (5, 21) lorsque le levier (28) qui le porte est déplacé dans un sens (44) correspondant à son éloignement du plateau (24), des moyens étant prévus pour permettre de déplacer les axes d'articulation (27) des leviers (28) dans un sens ou dans l'autre, parallèlement à l'axe (22c) du manche (22), tandis que d'autres moyens sont prévus pour faire pivoter les leviers (28) en position d'accrochage lorsqu'après leur engagement dans les encoches (33) ou lumières (34), leurs axes d'articulation (27) sont déplacés en direction du plateau (24) provoquant, de ce fait, une flexion de toutes les languettes (5, 21) en position effacée.

**6.** Moyen selon la revendication 5, caractérisé en ce que les moyens qui, dans l'appareil, permettent de déplacer les axes d'articulation (27) des leviers (28) à crochets (31, 32) dans les deux sens (35, 44), parallèlement à l'axe (22c) du manche (22), comprennent, en combinaison, une bague (26) supportant ces axes (28) et montée, axialement mobile, dans un évidement cylindrique (25a) d'une pièce cylindrique (25) coaxiale au plateau (24) et présentant des encoches radiales (29) dont chacune sert au logement et au guidage d'un levier (28) à crochets (31, 32), une tige de commande (38) coaxiale au plateau (24) et au manche (22) dans l'alésage (22d) duquel elle est logée, dont une extrémité antérieure (38a) est liée, axialement, par goupille (39) ou autre similaire, à la bague (26) supportant les axes d'articulation (27) des leviers (28) à crochets (31, 32), et dont l'extrémité postérieure (38b) est liée, axialement, à un anneau fileté (42) monté dans un alésage taraudé (43a) d'un volant de manoeuvre (43) porté par le manche (22), cet anneau fileté (42) étant, en outre, lié en rotation au manche (22), par exemple par une goupille (41) traversant l'anneau fileté (42), l'extrémité considérée (38b) de la tige de commande (38) et des lumières longitudinales (45) diamétralement opposées du manche tubulaire (22).

**7.** Moyen selon la revendication 5 ou 6, caractérisé en ce que les moyens qui, dans l'appareil, permettent de faire pivoter les leviers (28) à crochets (31, 32), de leur position effacée à leur position d'accrochage, comprennent, d'une part, un bossage (30) porté par chaque fond d'encoche radiale (29) servant au logement et au guidage d'un levier (28), auquel est associé un bossage opposé (40) porté par la face la plus interne radialement de chaque levier (28), ces bossages (30, 40) étant disposés de manière que le second (40) monte sur le premier (30) dès que, depuis leur position la plus éloignée du plateau (24), les axes d'articulation (27) des leviers (28) sont déplacés en direction (35) du plateau (24) et, d'autre part, un moyen à ressort annulaire (37) entourant les leviers (28) et agissant sur eux pour tendre constamment à les ramener en position effacée.

FIG.1

FIG.2

FIG.3

FIG.4

FIG.5

FIG_6

FIG_7

FIG_8

FIG_9

FIG_10

FIG_11

13

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | FR-A-2 416 004 (P.-D. SEJOURNE)<br>* Page 2, lignes 1-27; page 3, lignes 5-17; figures 1,4 * | 1-4 | A 61 F 2/34 |
| A | FR-A-2 266 491 (ATELIERS DE LA HAUTE-GARONNE-ETABLISSEMENTS AURIOL & CIE etc.)<br>* Page 3, ligne 34 - page 4, ligne 5; figures 3-4 * | 1-4 | |
| A | FR-A-2 617 040 (ETUDES ET RECHERCHES APPLIQUEES AND TRAITEMENTS ORTHOPEDIQUES)<br>* Page 1, ligne 33 - page 2, ligne 37; figures 1-2 * | 4 | |
| A | FR-A-2 595 562 (J.-L. RHENTER et al.)<br>* Page 1, lignes 13-34; page 5, lignes 16-29; figure 4 * | 4 | |
| A | WO-A-8 605 679 (Z. BADO et al.)<br>* Page 4, ligne 34 - page 5, ligne 19; page 7, lignes 4-8; page 12, lignes 9-28; figures 12-14 * | 5-7 | DOMAINES TECHNIQUES RECHERCHES (Int. Cl.5)<br><br>A 61 F |

Le présent rapport a été établi pour toutes les revendications

| Lieu de la recherche | Date d'achèvement de la recherche | Examinateur |
|---|---|---|
| LA HAYE | 16-07-1990 | NICE P.R. |

EPO FORM 1503 03.82 (P0402)